# EUROPÄISCHE PATENTSCHRIFT

(11) **EP 2 051 972 B1**
(45) Veröffentlichungstag und Bekanntmachung des Hinweises auf die Patenterteilung: **18.08.2010**
(21) Anmeldenummer: 08761344.4
(22) Anmeldetag: 24.06.2008
(51) Int. Cl.: C07D 307/06

(54) **VERFAHREN ZUR EINSTUFIGEN HERSTELLUNG VON 2-METHYLTETRAHYDROFURAN AUS FURFURAL AN ZWEI KATALYSATOREN IN STRUKTURIERTER SCHÜTTUNG**
PROCESS FOR ONE-STAGE PREPARATION OF 2-METHYLTETRAHYDROFURAN FROM FURFURAL OVER TWO CATALYSTS IN A STRUCTURED BED
PROCÉDÉ DE FABRICATION EN UNE ÉTAPE DE 2-MÉTHYLTÉTRAHYDROFURANE À PARTIR DE FURFURAL SUR DEUX CATALYSEURS DANS UN GARNISSAGE STRUCTURÉ

(30) Priorität: 02.07.2007 EP 07111508
(43) Veröffentlichungstag der Anmeldung: 29.04.2009
(73) Patentinhaber: BASF SE, 67056 Ludwigshafen (DE)
(72) Erfinder: WABNITZ, Tobias, 68169 Mannheim (DE); BREUNINGER, Daniel, 67240 Bobenheim-roxheim (DE); HEIMANN, Jens, 67550 Worms (DE); BACKES, Rene, 68623 Lampertheim (DE); PINKOS, Rolf, 67098 Bad Dürkheim (DE)
(86) Internationale Anmeldenummer: PCT/EP2008/058038
(87) Internationale Veröffentlichungsnummer: WO 2009/003881

(56) Entgegenhaltungen:
- WO-A-02/34697
- ZHENG ET AL: "Towards understanding the reaction pathway in vapour phase hydrogenation of furfural to 2-methylfuran" JOURNAL OF MOLECULAR CATALYSIS. A, CHEMICAL, ELSEVIER, AMSTERDAM, NL, Bd. 246, Nr. 1-2, 1. März 2006 (2006-03-01), Seiten 18-23, XP005299075 ISSN: 1381-1169

## Beschreibung

Die vorliegende Erfindung betrifft ein Verfahren zur einstufigen Herstellung von 2-Methyltetrahydrofuran aus Furfural an mindestens zwei Katalysatoren in strukturierter Schüttung.

2-Methyltetrahydrofuran (im folgenden 2-Me-THF) ist ein organisches Lösungsmittel mit hoher Lösekraft. 2-Me-THF wird als Ersatzlösungsmittel für chemische Synthesen für Tetrahydrofuran (im folgenden THF), von welchem es sich vorteilhaft durch seine geringere, mit zunehmender Temperatur abnehmende Wasserlöslichkeit unterscheidet, als Treibstoffzusatz, da es mit gängigen Treibstoffen auf Kohlenwasserstoff-Basis besser mischbar ist als alkoholische Zusätze, und als Comonomer für die Herstellung von Polyethern mit verbesserten Eigenschaften gegenüber den Homopolymeren verwendet.

2-Me-THF ist aus nachwachsenden Rohstoffen zugänglich. 2-Me-THF kann aus pflanzlichen Abfällen durch Aufschluss der enthaltenen Hemicellulosen zu Furfural und dessen Umsetzung zu 2-Me-THF erhalten werden und trägt so zu einer nachhaltigen Entwicklung bei.

Während die Herstellung von Furfural aus pflanzlichen, insbesondere landwirtschaftlichen Abfällen bekannt ist und einen hohen Ausarbeitungsstand erreicht hat, ist die Umsetzung von Furfural zu 2-Me-THF hingegen technisch noch nicht zufriedenstellend gelöst.

Die Elementarreaktionen der hydrierenden Umsetzung von Furfural sind bekannt und wurden von Zheng et al., Journal of Molecular Catalysis A: Chemical (2006), 246 (1-2), 18-23 detailliert beschrieben. Die Autoren halten 2-Methylfuran als Vorstufe bei der Herstellung von 2-Me-THF für notwendig und beschreiben Haupt- und Nebenreaktionen der Hydrierungen, einschließlich der Bildung von Kohlenmonoxid durch Decarbonylierung.

Obwohl 2-Me-THF häufig in geringen Mengen bei hydrierenden Umsetzungen von Furfural entsteht, gibt es nur wenige Veröffentlichung, in denen die direkte Umsetzung von Furfural zu 2-Me-THF beschrieben wird.

Kyosuke et al., J. Pharm. Soc. Jpn 66 (1946), 58 zeigen, dass die direkte Umsetzung von Furfural zu 2-Methyl-THF an Raney-Nickel-Katalysatoren bei 260°C nur geringe Mengen des Wertproduktes liefert. Als vorteilhaft empfehlen Kyosuke et al. stattdessen einen zweistufiger Prozess über Methylfuran als isoliertes Zwischenprodukt und die Verwendung von verschiedenen Katalysatoren für die beiden Stufen. So wird in der ersten Stufe Cu-Chromit nach Adkins und in der zweiten Stufe Raney-Nickel verwendet.

Proskuryakov et al., Trudy Leningradskogo Tekhnologicheskogo Instituta imeni Lensoveta (1958), 44, 3-5, beschreiben eine Ausbeute von maximal 42% 2-Me-THF bei der Umsetzung von Furfural an einer 1:1-Mischung aus einem Raney-Nickel- und einem Kupferchromitkatalysator in einem Autoklaven bei 220°C und 160 atm. Die Autoren zeigen, dass höhere 2-Me-THF-Anteile aufgrund von Nebenreaktionen zu Glykolen und anderen, nicht näher spezifizierten Ringöffnungsprodukten des Furfurals auf diesem Weg nicht erhalten werden können.

Als nachteilig wurde weiterhin die schwierige Isolierung und Reinigung von 2-Me-THF aus den erhaltenen Reaktionsaustragsgemischen erkannt, da die Nebenprodukte THF, 2-Pentanon und Wasser als Reinstoffe oder in Form ihrer Azeotrope Siedepunkte ähnlich dem des 2-Me-THF aufweisen. So liegen die Siedepunkte der Azeotrope Wasser/2-Me-THFbei 73°C, Wasser/THF bei 64°C und Wasser/2-Pentanon bei 84°C während die Reinstoffe THF bei 66°C, 2-Me-THF bei 80°C und 2-Pentanon bei 102 °C sieden.

Aus US-A 6,479,677 ist ein zweistufiges Verfahren zur Herstellung von 2-Me-THF unter Verwendung jeweils eines gesonderten Katalysators für jede Stufe bekannt. Jede Stufe wird in einem gesonderten Reaktor mit sich unterscheidenden Katalysatoren durchgeführt. Dieser Gasphasenprozess umfasst die Hydrierung von Furfural an einem Cu-Chromit-Katalysator zu Methylfuran, welches anschließend an einem NickelKatalysator zu 2-Me-THF umgesetzt wird.

Der offenbarte Prozess weist jedoch ein Reihe von Nachteilen auf. So werden für die einzelnen Stufen der Umsetzung verschiedene Reaktionsbedingungen benötigt, was die technische Ausführung erschwert und eine räumliche Trennung der einzelnen Reaktoren erforderlich macht. Die Zumischung von Wasserstoff ist für jeden Hydrierschritt getrennt erforderlich und ein großer Wasserstoffüberschuss, wie bei Gasphasenreaktionen oft erwünscht, wird nicht toleriert. Darüber hinaus führt die Bildung von Kohlenmonoxid, welches aus Furfural bei thermischer Belastung immer in geringen Mengen erfolgt, zu Deaktivierung des Nickel-Katalysators und zur Bildung von hochgiftigem, flüchtigem Ni(CO)₄. Durch die Aufpegelung kritischer Verunreinigungen wie Kohlenmonoxid wird die ökonomisch wünschenswerte Kreisgasfahrweise unmöglich.

Der vorliegenden Erfindung lag daher die Aufgabe zugrunde, ein Verfahren zur einstufigen Herstellung von 2-Methyltetrahydrofuran aus Furfural unter Verwendung spezifischer Katalysatoren ohne Isolierung beziehungsweise Aufreinigung von Zwischenprodukten zur Verfügung zu stellen, mit dessen Hilfe 2-Methyltetrahydrofuran insbesondere durch Umsetzung in einem Reaktor und in Kreislauffahrweise in guter Ausbeute und Reinheit erhalten werden kann.

Demgemäss betrifft die vorliegende Erfindung ein Verfahren zur einstufigen Hydrierung von Furfural mit einem wasserstoffenthaltenden Gas in Gegenwart einer strukturierten Schüttung aus mindestens einem Kupferkatalysator und mindestens einem Katalysator, der mindestens ein Edelmetall aus den Gruppen 8, 9 und/oder 10 des Periodensystems der Elemente aufgebracht auf einem Trägermaterial aufweist.

In einer bevorzugten Ausführungsform betrifft die vorliegende Erfindung ein Verfahren zur einstufigen Hydrierung von Furfural mit einem wasserstoffenthaltenden Gas in Gegenwart von zwei Katalysatoren, wobei der erste ein Kupferkatalysator und der zweite Katalysator als Aktivmetall mindestens ein Edelmetall aus den Gruppen 8, 9,10 des Periodensystems der Elemente, insbesondere Ruthenium, Rhodium, Iridium Gold, Palladium und/oder Platin, bevorzugt Palladium und/oder Platin, aufgebracht auf einem Trägermaterial aufweist, in strukturierter Schüttung.

Unter einstufig oder einstufiger Hydrierung wird in dieser Anmeldung ein Verfahren verstanden, das ausgehend von Furfural ohne Isolierung beziehungsweise Aufreinigung von Zwischenprodukten zum Endprodukt 2-Me-THF führt.

Als erster Katalysator der bevorzugten Ausführungsform wird der Katalysator bezeichnet, mit dem der Strom der Ausgangsstoffe, das Fufural und das wasserstoffhaltige Gas, bei Eintritt in den Reaktor zuerst in Berührung kommt, während der zweite Katalysator in Durchströmrichtung räumlich nach dem ersten Katalysator angeordnet ist und daher zeitlich erst nach diesem durchströmt wird.

### Erster Katalysator

Der erste Katalysator ist ein Kupferkatalysator, der bevorzugt neben Kupfer als zusätzliche Aktivmetalle ein oder mehrere Elemente der Gruppen 2, 6 und/oder 12 des Periodensystems der Elemente (Neue IUPAC Nomenklatur), insbesondere Chrom, Mangan, Zink, Barium enthält. Der Gesamtgehalt an den vorgenannten Aktivmetallen beträgt 10-100 Gew.-%, bevorzugt 15-80 Gew.-%, berechnet als Oxid. Der Kupfergehalt des Katalysators beträgt mindestens 10 Gew.-%, berechnet als Oxid, bevorzugt jedoch mindestens 15 Gew-%., besonders bevorzugt 15 bis 80 Gew-%.

Als erster Katalysator kommen Vollkatalysatoren in Betracht, bei denen die katalytisch wirkenden Metalle ohne Trägermaterialien vorliegen, entprechend einem Aktivmetallgehalt von 100 Gew.-%, sowie Fällungskatalysatoren oder Trägerkatalysatoren.

Ein bevorzugter Vollkatalysator ist der Katalysator der Zusammensetzung 24-26 Gew.-% Kupferoxid, 1 Gew.-% Chrom(VI)oxid, 1 Gew.-% Chrom(III)oxid, 0-4 Gew.-% Graphit, 65-67 Gew.-% Kupferchromit, der als Katalysator E 403-TU von der Firma Engelhard, Iselin USA, vertrieben wird.

Fällungskatalysatoren können hergestellt werden, indem man ihre katalytisch aktiven Komponenten aus deren Salzlösungen, insbesondere aus den Lösungen von deren Nitraten und/oder Acetaten, beispielsweise durch Zugabe von Lösungen von Alkalimetall- und/oder Erdalkalimetallhydroxid- und/oder Carbonat-Lösungen, z.B. als schwerlösliche Hydroxyde, Oxydhydrate, basische Salze oder Carbonate ausfällt, die erhaltenen Niederschläge anschließend trocknet und diese dann durch Calcinierung bei im allgemeinen 300 bis 700°C, insbesondere 400 bis 600°C, in die entsprechenden Oxide, Mischoxide und/oder gemischtvalentigen Oxide umwandelt, die durch eine Behandlung mit Wasserstoff oder mit Wasserstoff enthaltenden Gasen bei in der Regel 50 bis 700°C, insbesondere 100 bis 400°C, zu den betreffenden Metallen und/oder oxidischen Verbindungen niederer Oxidationsstufe reduziert und in die eigentliche, katalytisch aktive Form überführt werden. Dabei wird in der Regel so lange reduziert, bis kein Wasser mehr gebildet wird. Bei der Herstellung von Fällungskatalysatoren, die ein Trägermaterial enthalten, kann die Fällung der katalytisch aktiven Komponenten in Gegenwart des betreffenden Trägermaterials erfolgen. Die katalytisch aktiven Komponenten können vorteilhaft aber auch gleichzeitig mit dem Trägermaterial aus den betreffenden Salzlösungen gefällt werden. Bevorzugt werden im erfindungsgemäßen Verfahren Hydrierkatalysatoren eingesetzt, welche die Hydrierung katalysierenden Metalle oder Metallverbindungen auf einem Trägermaterial abgeschieden enthalten.

Außer den oben genannten Fällungskatalysatoren, welche außer den katalytisch aktiven Komponenten noch zusätzlich ein Trägermaterial enthalten, eignen sich für das erfindungsgemäße Verfahren im allgemeinen auch Trägerkatalysatoren, bei denen die katalytisch-hydrierend wirkende Komponenten, z.B. durch Imprägnierung, auf ein Trägermaterial aufgebracht worden sind.

Die Art der Aufbringung der katalytisch aktiven Metalle auf den Träger ist in der Regel nicht kritisch und kann auf verschiedenerlei Art und Weise bewerkstelligt werden. Die katalytisch aktiven Metalle können auf diesen Trägermaterialien z.B. durch Tränkung mit Lösungen oder Suspensionen der Salze oder Oxide der betreffenden Elemente, Trocknung und anschließende Reduktion der Metallverbindungen zu den betreffenden Metallen oder Verbindungen niederer Oxidationsstufe mittels eines Reduktionsmittels, vorzugsweise mit Wasserstoff oder komplexen Hydriden, aufgebracht werden. Eine andere Möglichkeit zur Aufbringung der katalytisch aktiven Metalle auf diese Träger besteht darin, die Träger mit Lösungen thermisch leicht zersetzbarer Salze, z.B. mit Nitraten, oder thermisch leicht zersetzbaren Komplexverbindungen, z.B. Carbonyl- oder Hydrido-Komplexen der katalytisch aktiven Metalle, zu imprägnieren und den so getränkten Träger zwecks thermischer Zersetzung der adsorbierten Metallverbindungen auf Temperaturen von 300 bis 600°C zu erhitzen. Diese thermische Zersetzung wird vorzugsweise unter einer Schutzgasatmosphäre vorgenommen. Geeignete Schutzgase sind z.B. Stickstoff, Kohlendioxid, Wasserstoff oder die Edelgase. Weiterhin können die katalytisch aktiven Metalle auf dem Katalysatorträger durch Aufdampfen oder durch Flammspritzen abgeschieden werden. Der Gehalt dieser Trägerkatalysatoren an den katalytisch aktiven Metallen ist prinzipiell für das Gelingen des erfindungsgemäßen Verfahrens nicht kritisch. Jedoch führen höhere Gehalte an katalytisch aktiven Metallen in der Regel zu höheren Raum-Zeit-Umsätzen als niedrigere Gehalte.

Im allgemeinen werden Trägerkatalysatoren verwendet, deren Gehalt an katalytisch aktiven Metallen, berechnet als Oxid, 10 bis 90 Gew.-%, vorzugsweise 15 bis 80 Gew.-%, bezogen auf den gesamten Katalysator, beträgt. Da sich diese Gehaltsangaben auf den gesamten Katalysator inklusive Trägermaterial beziehen, die unterschiedlichen Trägermaterialien jedoch sehr unterschiedliche spezifische Gewichte und spezifische Oberflächen haben, können diese Angaben aber auch unter- oder überschritten werden, ohne dass sich dies nachteilig auf das Ergebnis des erfindungsgemäßen Verfahrens auswirkt. Selbstverständlich können auch mehrere der katalytisch aktiven Metalle aus dem jeweiligen Trägermaterial aufgebracht sein.

Sowohl die Aktivierung der Fällungskatalysatoren als auch der Trägerkatalysatoren kann auch in situ zu Beginn der Reaktion durch den anwesenden Wasserstoff erfolgen, bevorzugt werden diese Katalysatoren jedoch vor ihrer Verwendung separat aktiviert.

Als Trägermaterialien können im allgemeinen die Oxide des Aluminiums, Zirkoniumdioxid, Siliciumdioxid, Magnesium und Calciumoxid verwendet werden. Selbstverständlich können auch Mischungen verschiedener Trägermaterialien als Träger für im erfindungsgemäßen Verfahren einsetzbaren Heterogenkatalysatoren dienen. Als für im erfindungsgemäßen Verfahren einsetzbarer erster Katalysator seien die folgenden beispielhaft genannt: 25 Gew.-% Kupferoxid, 1 Gew.-% Cr₂O₃ und 74 Gew.-% SiO₂.

### Zweiter Katalysator

Der zweite erfindungsgemäß verwendete Katalysator weist als als Aktivmetall mindestens ein Edelmetall aus den Gruppen 8, 9,10 des Periodensystems der Elemente , insbesondere Ruthenium, Rhodium, Iridium Gold, Palladium und/oder Platin, bevorzugt-Palladium und/oder Platin,besonders bevorzugt Palladium, auf einem Träger auf. Der zweite Katalysator kann zusätzlich Metalle der Gruppen 1, 2, 4 und 7 bis 12 des Periodensystems der Elemente aufweisen. Als Elemente der Gruppen 1 und 2 des Periodensystems der Elemente können insbesondere Natrium, Kalium, Calcium oder Magnesium verwendet werden. Bevorzugt weist er keine weiteren Aktivmetalle außer Palladium und Platin auf.

Die Auftragung der Aktivmetalle kann durch Tränken des Trägers in wässrigen Metallsalzlösungen, wie z.B. wässrigen Palladiumsalzlösungen, durch Aufsprühen entsprechender Metallsalzlösungen auf den Träger oder durch andere geeignete Verfahren erreicht werden. Als Metallsalze des Platins und Palladiums eignen sich die Nitrate, Nitrosylnitrate, Halogenide, Carbonate, Carboxylate, Acetylacetonate, Chloride, Chlorokomplexe oder Aminkomplexe der entsprechenden Metalle, wobei die Nitrate bevorzugt sind.

Bei Katalysatoren, die Palladium und Platin sowie gegebenenfalls noch weitere Aktivmetalle auf dem Träger aufweisen, können die Metallsalze bzw. Metallsalzlösungen gleichzeitig oder nacheinander aufgebracht werden.

Die mit der Metallsalzlösung beschichteten bzw. getränkten Träger werden anschließend, vorzugsweise bei Temperaturen zwischen 100°C und 150°C, getrocknet und wahlweise bei Temperaturen zwischen 200°C und 600°C, vorzugsweise zwischen 350°C und 450°C calciniert. Bei getrennter Auftränkung wird der Katalysator nach jedem Tränkschritt getrocknet und wahlweise calciniert, wie oben beschrieben. Die Reihenfolge, in der die Aktivkomponenten aufgetränkt werden, ist dabei frei wählbar.

Anschließend werden die beschichteten und getrockneten sowie wahlweise calcinierten Träger durch Behandlung in einem Gasstrom, der freien Wasserstoff enthält, bei Temperaturen zwischen ungefähr 30°C und ungefähr 600°C, vorzugsweise zwischen ungefähr 150°C und ungefähr 450°C aktiviert. Vorzugsweise besteht der Gasstrom aus 50 bis 100 Vol.-% H₂ und 0 bis 50 Vol.-% N₂.

Die Metallsalzlösung oder -lösungen werden in einer solchen Menge auf den oder die Träger aufgebracht, dass der Gesamtgehalt an Aktivmetall, jeweils bezogen auf das Gesamtgewicht des Katalysators, ungefähr 0,1 bis ungefähr 30 Gew.-%, vorzugsweise ungefähr 0,1 bis ungefähr 10 Gew.-%, weiter bevorzugt ungefähr 0,25 bis ungefähr 5 Gew.-%, und insbesondere ungefähr 0,5 bis ungefähr 2,5 Gew.-% beträgt.

Als Trägermetall verwendbar sind beispielsweise Aktivkohle, beispielsweise in Form des Handelsproduktes Supersorbon-Kohle der Firma Donau Carbon GmbH, 60388 Frankfurt am Main, Aluminiumoxid, Siliciumdioxid, Siliciumcarbid, Calciumoxid , Titandioxid und/oder Zirkoniumdioxid oder deren Gemische, wobei Aktivkohle vorzugsweise verwendet werden.

### Die Verfahrensführung

Das erfindungsgemäße Verfahren zeichnet sich durch eine strukturierte Schüttung des ersten und zweiten Katalysators aus.

Die erfindungsgemäße einstufige Hydrierung kann in einem oder mehreren, insbesondere zwei, drei, vier, fünf, sechs, sieben, acht Reaktoren durchgeführt werden. Bei Verwendung mehrerer Reaktoren kann sich der erste Katalysator in einem ersten Reaktor oder in einer ersten Gruppe aus mindestens zwei Reaktoren befinden und die Schüttung des zweiten Katalysators befindet sich im zweiten Reaktor oder in der zweiten Gruppe aus mindestens zwei Reaktoren. Bevorzugt wird die einstufige Hydrierung jedoch in einem Reaktor durchgeführt.

Das Reaktionsgemisch durchströmt den Reaktor oder die Reaktoren bevorzugt jeweils von oben nach unten. Dabei wird bei Verwendung eines Reaktors und entsprechend bei Verwendung mehrere Reaktoren für die 20 bis 80 % der Gesamthöhe der Katalysatorschüttung, bevorzugt die oberen 40 bis 60 %, der Gesamthöhe der Schüttung nach dem Reaktorkopf des einzigen bzw. des ersten Reaktors der erste Katalysator (Kupferkatalysator) verwendet. Die verbleibende Schütthöhe wird dann von dem zweiten Katalysator (Palladium und/ oder Platin-Katalysator) gebildet. Wird der Reaktor oder die Reaktoren von unten nach oben durchströmt, werden die Katalysatoren in umgekehrter Reihenfolge angeordnet.

Bei Verwendung eines einzelnen Reaktors kann es vorteilhaft sein, den aus dem ersten Katalysator gebildeten Teil der Schüttung von dem aus dem zweiten Katalysator gebildeten Teil durch das Einbringung einer Zwischenschicht aus inertem Material zu trennen. Als inerte Zwischenschicht sind beispielsweise Glasringe oder Metallringe geeignet. Die Hydrierung des Furfurals zum 2-Me-THF kann daher vorteilhaft einstufig in einem Reaktor durchgeführt werden.

Im Rahmen des erfindungsgemäßen Verfahrens kann die Hydrierung in der Gas -oder der Flüssigphase durchgeführt werden, bevorzugt wird in der Gasphase gearbeitet. Im allgemeinen wird das Verfahren in der Gasphase bei einer Temperatur von ungefähr 150 bis 250°C, vorzugsweise ungefähr 190 bis 230°C durchgeführt. Die dabei verwendeten Drücke liegen in der Regel bei 1 bis 10 bar absolut, vorzugsweise ungefähr 1 bis 3 bar abs. Der Druck in dieser Anmeldung ist als Gesamtdruck oder absoluter (abs.) Druck angegeben.

In der Flüssigphase wird das erfindungsgemäße Verfahren im allgemeinen bei 150 bis 250°C bei Drücken von 1 bis 250 bar abs., bevorzugt bei 20 bis 200 bar abs., durchgeführt.

Neben dem Kupferkatalysator und dem Edelmetallkatalysator, die zur Ausführung des erfindungsgemäßen Verfahrens erforderlich und als erster und zweiter Katalysator bezeichnet werden, können sich weitere Katalysatoren im Reaktor bzw. in den Reaktoren befinden. Diese Katalysatoren können beispielsweise dazu dienen, die Produktqualität zu verbessern, indem Verunreinigungen entfernt werden oder Nebenprodukte umgesetzt werden. Beispielsweise können schwefelhaltige Komponenten, die in Furfural in der Regel in geringer Menge vorhanden sind, durch Behandlung mit Entschwefelungskatalysatoren oder Adsorbentien auf Basis von Kupfer und/oder Molybdän- und/oder Zinkoxiden entfernt werden. Als Nebenprodukt beim Umgang mit Furfural fallen immer geringe Mengen Kohlenmonoxid an. Dieses Nebenprodukt kann beispielsweise durch Hydrierung an Kupfer und/oder Rutheniumkatalysatoren zu Methanol umgesetzt werden und so entfernt werden, analog lassen sich ketonische und aldehydische Nebenprodukte zu Alkoholen umsetzen. Katalysatoren, die diese und ähnliche Funktionen erfüllen, können sich, zusätzlich zu den für die Durchführung des erfindungsgemäßen Verfahrens zwingend erforderlichen Katalysatoren, an jeder Stelle im Reaktor befinden. Sie können sowohl vor dem Kupferkatalysator als auch nach dem Edelmetallkatalysator als auch zwischen den beiden Katalysatoren eingebaut werden. Es ist ebenfalls denkbar, solche Katalysatoren in homogener Mischung einzusetzen, solange dabei die strukturierte Schüttung des Kupferkatalysators und des Edelmetallkatalysators nicht aufgehoben wird.

Das erfindungsgemäße Verfahren kann entweder kontinuierlich oder diskontinuierlich durchgeführt werden, wobei die kontinuierliche Verfahrensdurchführung bevorzugt ist. Bei der kontinuierlichen Verfahrensführung beträgt die Menge des zur Hydrierung Furfurals ungefähr 0,05 bis ungefähr 3 kg pro Liter Katalysator pro Stunde, weiter bevorzugt ungefähr 0,1 bis ungefähr 1 kg pro Liter Katalysator pro Stunde.

Als Hydriergase können beliebige Gase verwendet werden, die freien Wasserstoff enthalten und keine schädlichen Mengen an Katalysatorgiften, wie beispielsweise CO, aufweisen. Beispielsweise können Reformerabgase verwendet werden. Vorzugsweise wird reiner Wasserstoff als Hydriergas verwendet. Es ist jedoch auch möglich zusätzlich inerte Trägergase wie Wasserdampf oder Stickstoff zu verwenden.

### Wassstoff/Furfural-Molverhältnis

Das Mischungsverhältnis von Wasserstoff und Furfural ist nicht kritisch, solange ausreichende Mengen Wasserstoff (4 Äquivalente) für die Umsetzung von Furfural zu 2-Methyl-THF zur Verfügung stehen. Ein Wasserstoffüberschuss ist möglich. Bei der kontinuierlichen Verfahrensführung beträgt das Wasserstoff/Furfural-Verhältnis am Reaktoreingang 4:1 bis 500:1, bevorzugt 5:1 bis 250:1, besonders bevorzugt 10:1 bis 100:1.

In der Flüssigphase kann die erfindungsgemäße Hydrierung in Ab- oder Anwesenheit eines Lösungs- oder Verdünnungsmittels durchgeführt werden, d.h. es ist nicht erforderlich, die Hydrierung in Lösung durchzuführen.

Es kann jedoch ein Lösungs- oder Verdünnungsmittel eingesetzt. Als Lösungs- oder Verdünnungsmittel kann jedes geeignete Lösungsmittel- oder Verdünnungsmittel eingesetzt werden. Die Auswahl ist dabei nicht kritisch, solange das eingesetzte Lösungs- oder Verdünnungsmittel in der Lage ist, mit dem zu hydrierenden Furfural eine homogene Lösung zu bilden.

Beispiele geeigneter Lösungs- oder Verdünnungsmittel schließen die folgenden ein: Geradkettige oder cyclische Ether, wie beispielsweise Tetrahydrofuran oder Dioxan, sowie aliphatische Alkohole, in denen der Alkylrest vorzugsweise 1 bis 10 Kohlenstoffatome, insbesondere 3 bis 6 Kohlenstoffatome aufweist.

Die Menge des eingesetzten Lösungs- oder Verdünnungsmittels ist nicht in besonderer Weise beschränkt und kann je nach Bedarf frei gewählt werden, wobei jedoch solche Mengen bevorzugt sind, die zu einer 10 bis 70 gew.-%igen Lösung des zur Hydrierung vorgesehenen Furfurals führen.

Weiterhin wird kann der Hydrierreaktor bei der Durchführung der Hydrierung in der Flüssigphase im geraden Durchgang, das heißt ohne Produktrückführung, oder im Umlauf (Kreislauf), das heißt ein Teil des den Reaktor verlassenden Hydriergemischs wird im Kreislauf geführt, betrieben werden.

Bei Durchführung der erfindungsgemäßen Hydrierung in der Gasphase werden die Reaktionsprodukte nach Verlassen des Reaktors vollständig kondensiert und abgetrennt. Die gasförmigen Anteile, Wasserstoff und eventuell verwendetes zusätzliches Trägergas, werden teilweise im Umlauf in den Reaktor zurückzufahren (Kreisgasfahrweise). Bei der bevorzugten Kreisgasfahrweise beträgt das Verhältnis von Kreisgas- zu Frischgasvolumina mindestens 1:1, bevorzugt mindestens 5:1, besonders bevorzugt mindestens 10:1.

Als Reaktoren kommen Festbettreaktoren wie beispielsweise Rohrbündelreaktoren in Betracht. Die Wahl des Reaktortyps ist an sich nicht kritisch solange die Katalysatoranordnung in der Schüttung, das heißt die Reihenfolge in der das Reaktionsgemisch die Katalysatoren durchströmt, nicht verändert wird. In der Flüssigfahrweise sind Fließbettreaktoren verwendbar, wenn die Betten der beiden Katalysatortypen so angeordnet sind, dass eine Vermischung des ersten und zweiten Katalysators beim Betrieb des Reaktors ausgeschlossen ist.

Die Reaktionsausträge der erfindungsgemäßen Hydrierung werden in an sich bekannter Weise, bevorzugt jedoch durch Abkühlung in einem Wärmetauscher auf 0 bis 80°C kondensiert. Nach der Kondensation stellt sich eine Phasentrennung ein. Die untere Phase besteht zu über 90 % aus Wasser, während die obere Phase neben dem gewünschten Produkt 2-Me-THF nur geringe Mengen an durch eine gegebenenfalls nachfolgende Reindestillation gut abtrennbaren Nebenprodukten aufweist. 2-Methyltetrahydrofuran (2-Me-THF) wird durch das erfindungsgemäße Verfahren in sehr guter Ausbeute und Reinheit erhalten. Die Phasentrennung kann bei Umgebungstemperatur erfolgen. Die Reaktionsausträge werden jedoch bevorzugt bei 60 °C kondensiert, da bei dieser Temperatur die Mischbarkeit von 2-Methyl-THF und Wasser besonders gering ist.

Im folgenden soll nunmehr das erfindungsgemäße Verfahren anhand einiger Ausführungsbeispiele näher erläutert werden.

### Beispiele

### Herstellungsbeispiel Katalysator

4 kg Supersorbon-Kohle (4 mm Stränge, Hersteller Donau Carbon GmbH) wurden in einer Imprägniertrommel vorgelegt und mit 2,8 kg einer bezogen auf Palladium 7,2 gew.-%igen wässrigen Lösung von Palladium(II)-Nitrat bei Raumtemperatur mit Hilfe einer feinen Düse (1 mm) besprüht. Die Flüssigkeit wurde vollständig in den Poren des Kohleträgers aufgenommen. Das Material wurde anschließend 40 Stunden bei 100 °C im Wärmeschrank getrocknet.

Anschließend wurde der getrocknete Katalysator bei 200°C im Wasserstrom aktiviert (reduziert). Der so hergestellte Katalysator enthielt 5 Gew.-% Palladium, bezogen auf das Gewicht des Katalysators.

### Analytik der Hydrierprodukte

Die Reaktionsprodukte 2-Me-THF, 2-Pentanon, 3-Pentanon, 1-Pentanol, THF, Furan und Methylfuran sowie das Ausgangsmaterial Furfural wurden gaschromatographisch analysiert. Die Mischungen wurden dazu verdünnt mit Methanol oder Aceton (Verdünnung von1:10 bis 1:100) oder unverdünnt in den GC-Chromatographen (Firma HP, Trägergas: Wasserstoff) auf eine 30 m DB1-Säule (Firma J+W) gespritzt und bei Ofentemperaturen von 60°C bis 300°C (Aufheizrate 8 Kelvin pro Minute bis 220°C, dann 20 Kelvin pro Minute bis 300°C) mit einem Flammenionisationsdetektor (Temperatur: 290°C) analysiert. Die Reinheit wurde durch Integration der Signale des Chromatogramms bestimmt.

### Beispiel 1

In einer Anlage zur kontinuierlichen Hydrierung bestehend aus einem Verdampfer, einem ölbeheizten 3,81-Doppelmantelrohrreaktor sowie einem Abscheider und einem Kreisgaskompressor wurde Furfural kontinuierlich an Festbettkatalysatoren in der Gasphase hydriert.

Der Rohrreaktor wurde mit 450 g eines Pd-Katalysators (5 % Pd/Supersorbon Kohle, 4 mm Stränge), dann oben mit 530 g des Kupferchromit-Katalysators E 403-TU der Firma Engelhard Iselin; USA jetzt BASF (24-26 Gew.-% Kupferoxid, 1 Gew.-% Chrom(VI)oxid, 1 Gew.-% Chrom(III)oxid, 0-4 Gew.-% Graphit, 65-67 Gew.-% Kupferchromit ,Tabletten 3 mm x 3 mm) gefüllt.

Der Rohrreaktor wurde von oben nach unten durchströmt. Die Katalysatoren wurden nach dem Fachmann bekannter Methode drucklos mit Stickstoff/Wasserstoffgemischen bei 200°C aktiviert, so dass der Gehalt an Wasserstoff im Mischgas langsam von 0 bis auf 100 % gesteigert wurde. Anschließend wurde die Anlage mit Wasserstoff auf 4 bar aufgepresst, Frischgas Wasserstoff auf 450 NL/h eingestellt, der Verdampfer auf 230°C, der Reaktor auf 220°C temperiert und das Kreisgas in Betrieb genommen. In den Verdampfer wurden 400 g/h einstufig destilliertes Furfural gefördert. Während der Hydrierung wurde das Kreisgas auf 240 g/h eingestellt, 83 NL/h Abgas wurden der Verbrennung zugeführt. Unter diesen Bedingungen wurde über 440 h Furfural zu > 99 % umgesetzt, die Selektivität zu 2-MeTHF war > 80 %. Die obere Phase des zweiphasigen Austrags hatte die folgende Zusammensetzung: Furan, 2-Methylfuran, THF je < 0,5 Gew.-%, 2-MeTHF 84 Gew.-%, 2-Pentanon 2,4 Gew.-%, 2-Pentanol 1,3 Gew.-%, 1-Pentanol 4 Gew.-%, Methyl-gamma-Butyrolacton 5 Gew.-%, Rest zu 100 % nicht identifizierte Nebenprodukte. Die Nebenprodukte konnten mittels Destillation nach dem Stand der Technik entfernt werden, so dass das gewünschte Produkt 2-MeTHF in einer Reinheit von > 99 % erhalten wurde.

### Beispiel 2

In einem elektrisch beheizbaren, vertikal installierten Quarzrohr als Reaktor (Innendurchmesser 2,5 cm, Länge 60 cm) wurden 50 mL (21 g) eines Palladium-Kohle-Katalysators gemäß Beispiel 1 (5 % Pd auf Supersorbon-K, 4 mm Stränge) mit 10 mL Quarzglasringen (zur Trennung der Katalysatorschichten) und 50 mL (30 g) eines Kupfer-Chromit-Katalysators E 403-TU der Firma Engelhard Iselin; USA (24-26 Gew.-% Kupferoxid, 1 Gew.-% Chrom(VI)oxid, 1 Gew.-% Chrom(III)oxid, 0-4 Gew.-% Graphit, 65 bis 67 Gew.-% Kupferchromit ,Tabletten 3 mm x 3 mm) überschichtet und mit weiteren 100 mL Quarzglasringen (als Verdampfungsstrecke) bedeckt.

Das Quarzrohr mit dieser strukturierten Katalysator/Quarzglasring-Schüttung wurde mit einem Gas- und einem Flüssigkeitszulaufrohr versehen und so installiert, dass die Reaktionsprodukte in einem Glaswendelkühler kondensiert und gesammelt wurden. Der Reaktor wurde von oben nach unten durchströmt, das heißt, die Reaktionsmischung durchströmte zunächst den Kupferchromit-Katalysator, anschließend den Pd-Katalysator.

Die Katalysatoren wurden durch Behandlung mit Wasserstoff, zunächst verdünnt in einem Stickstoffstrom, später in Reinform, jeweils bei Umgebungsdruck und 200°C für 2 h aktiviert. Dann wurden dann 5 mL/h (ca. 5,8 g/h) Furfural bei einem Wasserstoffstrom von 15 L/h und Reaktortemperaturen von a) 175, b) 200, c) 225 und bei d) 250°C und Umgebungsdruck umgesetzt. Der Umsatz des Edukts (Furfural) war bei allen vorgenannten Temperaturen a) bis d) vollständig. Die Reaktionsausträge wurden kondensiert und in Folge der Kondensation trennte sich der Reaktionsaustrag in zwei Phasen an. Die untere Phase, etwa <1/5 der Gesamtmenge des jeweiligen Reaktionsaustrags, bestand zu >90 % aus Wasser und wurde verworfen. Die obere Phase wies in Abhängigkeit von der Reaktionstemperatur folgende, gaschromatographisch bestimmte Zusammensetzungen auf:

**Tabelle 1**

| Bsp. | T °C | 2-Me-THF Gew.-% | 2-Pentanon | 3-Pentanon | 1-Pentanol | THF | Methyl -furan | Furan |
|---|---|---|---|---|---|---|---|---|
| 2 a | 175 | 91 | 0,5 | <0,5 | 2 | <0,5 | <0,5 | <0,5 |
| 2 b | 200 | 91 | 2 | 1 | 2 | 1 | <0,5 | <0,5 |
| 2 c | 225 | 86 | 4 | 2 | 1 | 2 | <0,5 | 1 |
| 2 d | 250 | 82 | 5 | <0,5 | 1 | 4 | 0,5 | 0,5 |

Neben den organischen Anteilen enthielten die oberen Phasen der Austräge zusätzlich 2 bis 8% Wasser.

Die obere Phase, die das Reaktionsprodukt 2-Me-THF enthielt, wurde in einer Füllkörperkolonne einer von Länge 1,2 m, einem Innendurchmesser von 2,5 cm mit 3x3 mm V2A-Metallringe als Füllkörper destilliert und das Produkt 2-Me-THF konnte in >99 % Reinheit gewonnen werden.

### Beispiel 3

Analog Beispiel 2 wurde ein Kupferkatalysator mit mineralischem Träger (Zusammensetzung im nichtaktivierten Zustand 25 % CuO / 1 %Cr₂O₃ / 74 % SiO₂) und ein Palladium-Kohle-Katalysator (5 % Pd auf Supersorbon-K, 4 mm Stränge) in den Reaktor eingebaut. Bei 225°C wurde folgende Zusammensetzung des Reaktionsaustrags erhalten:
85 % 2-Me-THF, 5 % 2-Pentanon, <0,5 % 3-Pentanon, <0,5 % 1-Pentanol, 1 % 2-Pentanol, 4 % THF, 0,5% Furan, <0,5 % Methylfuran sowie weitere, nicht identifizierte Produkte.

### Beispiel 4

Analog Beispiel 2 wurden 24,2 g eines Kupferkatalysators mit mineralischem Träger der Zusammensetzung im nichtaktivierten Zustand 25 % CuO / %Cr₂O₃ / 74 % SiO₂ und 28,1 g eines Platin-Kohle-Katalysators (5 % Pt auf 4 mm Strängen Supersorbon K) in den Reaktor eingebaut. Bei 225°C wurde folgende Zusammensetzung des Reaktionsaustrags erhalten: 82 % 2-Me-THF, 4 % 2-Pentanon, 1 % 3-Pentanon, <0,5 % 1-Pentanol, 1 % 2-Pentanol, 3 % THF, 0,5 % Furan, 1 % Methylfuran sowie weitere, nicht identifizierte Produkte.

## Patentansprüche

1. Verfahren zur Herstellung von 2-Methyltetrahydrofuran durch einstufige Hydrierung von Furfural mit einem wasserstoffenthaltenden Gas in Gegenwart einer strukturierten Schüttung aus mindestens einem Kupferkatalysator und mindestens einem Katalysator, der mindestens ein Edelmetall aus den Gruppen 8, 9 und/oder 10 des Periodensystems der Elemente aufgebracht auf einem Trägermaterial aufweist.

2. Verfahren zur Herstellung von 2-Methyltetrahydrofuran durch einstufige Hydrierung von Furfural nach Anspruch 1, **dadurch gekennzeichnet, dass** in Gegenwart von zwei Katalysatoren in strukturierter Schüttung hydriert wird.

3. Verfahren nach Anspruch 2, **dadurch gekennzeichnet, dass** der Strom der Ausgangsstoffe der Hydrierung zuerst den Kupferkatalysator (erster Katalysator) und so dann den Edelmetall enthaltenden Trägerkatalysator (zweiter Katalysator) durchströmt.

4. Verfahren nach Anspruch 2 oder 3, **dadurch gekennzeichnet, dass** die strukturierte Schüttung der beiden Katalysatoren sich in einem oder mehreren Reaktoren befindet.

5. Verfahren nach einem der Ansprüche 1 bis 4, **dadurch gekennzeichnet, dass** der erste Katalysator (Kupferkatalysator) als Aktivmetalle Kupfer und ein oder mehrere Elemente der Gruppen 2, 6 und/oder 12 des Periodensystems der Elemente aufweist.

6. Verfahren nach Anspruch 2 bis 5, **dadurch gekennzeichnet, dass** der erste Katalysator neben Kupfer Chrom, Mangan, Zink und/oder Barium enthält.

7. Verfahren nach einem der Ansprüche 2 bis 6, **dadurch gekennzeichnet, dass** der Kupfergehalt des ersten Katalysators mindestens 10 Gew.-% beträgt.

8. Verfahren nach einem der Ansprüche 2 bis 7, **dadurch gekennzeichnet, dass** der zweite Katalysator Palladium und/oder Platin auf einem Träger aufweist.

9. Verfahren nach Anspruch 8, **dadurch gekennzeichnet, dass** der zweite Katalysator 0,1 bis ungefähr 30 Gew.-% Palladium und/oder Platin auf einem Träger aufweist.

10. Verfahren nach Anspruch 8 oder 9, **dadurch gekennzeichnet, dass** der zweite Katalysator mindestens ein Element ausgewählt aus den Gruppen 1, 2, 4 und 7 bis 12 des Periodensystems der Elemente aufweist.

11. Verfahren nach einem der Ansprüche 8 bis 10, **dadurch gekennzeichnet, dass** Aktivkohle, Aluminiumoxid, Siliciumdioxid, Siliciumcarbid, Calciumoxid, Titandioxid und/oder Zirkoniumdioxid oder deren Gemische als Träger des zweiten Katalysators verwendet werden.

12. Verfahren nach einem der Ansprüche 1 bis 11, **dadurch gekennzeichnet, dass** der erste Katalysator 20 bis 80 % der Gesamthöhe der Katalysatorschüttung der Gesamthöhe der Schüttung nach dem Reaktorkopf bildet.

13. Verfahren nach einem der Ansprüche 1 bis 12, **dadurch gekennzeichnet, dass** das Verfahren in der Flüssigphase bei einer Temperatur von 150 bis 250 C bei 20 bis 200 bar absolut durchgeführt wird.

14. Verfahren nach Anspruch 13, **dadurch gekennzeichnet, dass** das Verfahren in An- oder Abwesenheit eines Lösungsmittels durchgeführt wird.

15. Verfahren nach einem der Ansprüche 1 bis 12, **dadurch gekennzeichnet, dass** das Verfahren in der Gasphase bei einer Temperatur von 150 bis 250 C bei 1 bis 10 bar absolut durchgeführt wird.

16. Verfahren nach einem der Ansprüche 1 bis 15, **dadurch gekennzeichnet, dass** das Verfahren in Kreisgas oder Kreislauffahrweise durchgeführt wird.

## Claims

1. A process for preparing 2-methyltetrahydrofuran by one-stage hydrogenation of furfural with a hydrogen-comprising gas in the presence of a structured bed of at least one copper catalyst and at least one catalyst which composes at least one noble metal from stoups 8, 9 and/or 10 of the periodic of the elements applied on a support material.

2. The process for preparing 2-methyltetrahydrofuran by one-stage hydrogenation of furfural according claim 1, wherein hydrogenation is effected in the presence of two catalysts in the structured bed.

3. The process according to claim 2, wherein the stream of the staring materials of the hydrogenation flows first through the copper catalyst (first catalyst) and then through the supported catalyst comprising noble metal (second catalyst).

4. The process to claim 2 or 3, wherein the structured bed of the two catalysts is disposed in one or more reactors.

5. The process according to any of claims 1 to 4, wherein the first catalyst (copper catalyst) has, as active metals, copper and one or more elements from groups 2, 6 and/or 12 of the period table of the elements.

6. The process according to claims 2 to 5, wherein the first catalyst, as well as copper, comprises chromium, manganese, zinc and/or barium.

7. The process according to any of claims 2 to 6, wherein the copper content of the first catalyst is at least 10% by weight.

8. The process according to any of claims 2 to 7, wherein the second catalyst has palladium and/or platinum on a support.

9. The process according to claim 8, wherein the second catalyst comprises from 0.1 to about 30% by weight of palladium and/or platinum on a support.

10. The process according to claim 8 or 9, wherein the second catalyst comprises at least one element selected from groups 1, 2, 4 and 7 to 12 of the penodic table of the elements.

11. The process according to any of claims 8 to 10, wherein activated carbon, aluminum oxide, silicon dioxide, silicon carbide, calcium oxide, titanium dioxide and/or zirconium dioxide or mixtures thereof are used as the support of the second catalyst.

12. The process according to any of claims 1 to 11, wherein the first catalyst forms from 20 to 80% of the total height of the catalyst bed of the total height of the bed downstream of the top of the reactor.

13. The process according to any of claims 1 to 12, which is performed in the liquid phase at a temperature of from 150 to 250°C as from 20 to 200 bar absolute.

14. The process according to claim 13, which is performed in the presence or absence of a solvent.

15. The process according to any of claims 1 to 12, which is performed in the gas phase at a temperature of from 150 to 250°C at from 1 to 10 bar absolute.

16. The process according to any of claims 1 to 15, which is performed in cycle gas or circulation mode.

## Revendications

1. Procédé pour la préparation de 2-méthyltétrahydrofuranne par hydrogénation en une étape de furfural avec un gaz contenant de l'hydrogène, en présence d'un lit structuré d'au moins un catalyseur au cuivre et d'au moins un catalyseur qui contient un métal noble choisi dans les groupes 8, 9 et/ou 10 du système périodique des éléments, appliqué sur une matière de support.

2. Procédé pour la préparation de 2-méthyl tétrahydrofuranne par hydrogénation en une étape de furfural selon la revendication 1, **caractérisé en ce qu'**on effectue l'hydrogénation en présence de deux catalyseurs en lit structuré.

3. Procédé selon la revendication 2, **caractérisé en ce que** le courant des produits de départ de l'hydrogénation traverse d'abord le catalyseur au cuivre (premier catalyseur) et ensuite le catalyseur contenant un métal noble, fixé sur support (deuxième catalyseur).

4. Procédé selon la revendication 2 ou 3, **caractérisé en ce que** le lit structuré des deux catalyseurs se trouve dans un ou plusieurs réacteurs.

5. Procédé selon l'une quelconque des revendications 1 à 4, **caractérisé en ce que** le premier catalyseur (catalyseur au cuivre) comporte en tant que métaux actifs du cuivre et un ou plusieurs éléments des groupes 2, 6 et/ou 12 du système périodique des éléments.

6. Procédé scion l'une quelconque des revendications 2 à 5, **caractérisé en ce qu'**outre du cuivre le premier catalyseur contient du chrome, du manganèse, du zinc et/ou du baryum.

7. Procédé selon l'une quelconque des revendications 2 à 6, **caractérisé en ce que** la teneur en cuivre du premier catalyseur est d'au moins 10 % en poids.

8. Procédé selon l'une quelconque des revendications 2 à 7, **caractérisé en ce que** le deuxième catalyseur comporte du palladium, et/ou du platine sur un support.

9. Procédé selon la revendication 8, **caractérisé en ce que** le deuxième catalyseur comporte de 0,1 à environ 30 % en poids de palladium et/ou de platine sur un support.

10. Procédé selon la revendication 8 ou 9, **caractérisé en ce que** le deuxième catalyseur comprend au moins un élément choisi dans les groupes 1, 2, 4 et 7 à 12 du système périodique des éléments.

11. Procédé selon l'une quelconque des revendications 8 à 10, **caractérisé en ce que** comme support du deuxième catalyseur on utilise du charbon actif, de l'oxyde d'aluminium, du dioxyde de silicium, du carbure de silicium, de l'oxyde de calcium, du dioxyde de titane et/ou du dioxyde de zirconium ou des mélanges de ceux-ci.

12. Procédé selon l'une quelconque des revendications 1 à 11, **caractérisé en ce que** le premier catalyseur constitue de 20 à 80 % de la hauteur totale du lit de catalyseur de la hauteur totale du lit vers la tête du réacteur.

13. Procédé selon l'une quelconque des revendications 1 à 12, **caractérisé en ce que** le procédé est effectué dans la phase liquide à une température de 150 à 250 °C sous une pression absolue de 20 à 200 bars.

14. Procédé selon la revendication 13, **caractérisé en ce que** le procédé est effectué en présence ou en absence d'un solvant.

15. Procédé selon l'une quelconque des revendications 1 à 12, **caractérisé, en ce que** le procédé est effectué dans la phase gazeuse à une température de 150 à 250 °C sous une pression absolue de 1 à 10 bars.

16. procédé selon l'une quelconque des revendications 1 à 15, **caractérisé en ce que** le procédé est effectué en mode à circuit fermé ou à gaz en recirculation.
